# EUROPEAN PATENT APPLICATION

(11) **EP 3 508 119 A1**
(43) Date of publication of application: **10.07.2019**
(21) Application number: 16915073.7
(22) Date of filing: 30.08.2016
(51) Int. Cl.: A61B 5/11

(54) **ACTIVITY AMOUNT PROCESSING DEVICE, ACTIVITY AMOUNT PROCESSING METHOD, AND ACTIVITY AMOUNT PROCESSING PROGRAM**

(71) Applicant: Fujitsu Limited, Kawasaki-shi, Kanagawa 211-8588 (JP)
(72) Inventor: YAMAJI, Takayuki, Kawasaki-shi Kanagawa 211-8588 (JP); MASUDA, Yuta, Kawasaki-shi Kanagawa 211-8588 (JP)
(74) Representative: Hoffmann Eitle
(86) International application number: PCT/JP2016/075316
(87) International publication number: WO 2018/042512

(57) **Abstract**

In the present invention, activity amount data corresponding to activity of a test subject measured by an activity amount meter is acquired, a biorhythm of the test subject is detected from a result of frequency analysis of a time waveform of the activity amount data, and information on an action recommended to the test subject with respect to the detected biorhythm is visualized.

## Description

### Technical Field

The present invention relates to an activity amount processing device, an activity amount processing method, and an activity amount processing program.

### Background Art

There exists a technology configured to detect or estimate a physiological rhythm (may also be referred to as "biorhythm") of a biological body. For example, a technique configured to measure the rectal temperature of a test subject for equal to or more than 24 hours and estimate a biorhythm curve, a technique configured to measure the heartbeat of a test subject for equal to or more than 24 hours and estimate a biorhythm curve, and the like are known.

### Citation List

### Patent Literature

PTL 1: Japanese Laid-open Patent Publication No. 6-189914
PTL 2: Japanese Laid-open Patent Publication No. 6-217946
PTL 3: Japanese Laid-open Patent Publication No. 2005-198829
PTL 4: Japanese Laid-open Patent Publication No. 6-217946
PTL 5: Japanese Laid-open Patent Publication No. 2012-239799
PTL 6: International Publication Pamphlet No. WO 2012/011318
PTL 7: Japanese Laid-open Patent Publication No. 9-313492
PTL 8: Japanese Laid-open Patent Publication No. 2015-228911

### Summary of Invention

### Technical Problem

A test subject sometimes wants to know, with respect to his or her detected biorhythm, information indicating what types of actions regarding everyday life, sleep, and the like are preferable to be taken in order to maintain the biorhythm in a normal state.

In an aspect, an object of the present disclosure is to provide a test subject with information such as advice with regard to actions to be taken in accordance with the detected biorhythm of the test subject.

### Solution to Problem

In an aspect, an activity amount processing device may include an acquisition unit and a processing unit. The acquisition unit may acquire activity amount data corresponding to activity of a test subject measured by an activity amount meter. The processing unit may detect a biorhythm of the test subject from a result of frequency analysis of a time waveform of the activity amount data, and may visualize information on an action recommended to the test subject with respect to the detected biorhythm.

Further, in an aspect, an activity amount processing method may include: acquiring activity amount data corresponding to activity of a test subject measured by an activity amount meter; detecting a biorhythm of the test subject from a result of frequency analysis of a time waveform of the activity amount data; and visualizing information on an action recommended to the test subject with respect to the detected biorhythm.

Further, in an aspect, an activity amount processing program may cause a computer to execute a process including: acquiring activity amount data corresponding to activity of a test subject measured by an activity amount meter; detecting a biorhythm of the test subject from a result of frequency analysis of a time waveform of the activity amount data; and visualizing information on an action recommended to the test subject with respect to the detected biorhythm. Advantageous Effects of Invention

As an aspect, it is possible to provide a test subject with information such as advice on an action to be taken in accordance with the detected biorhythm of the test subject.

### Brief Description of Drawings

FIG. 1 is a block diagram illustrating a configuration example of an information processing system according to an embodiment.
FIG. 2 is a block diagram illustrating a configuration example of an activity amount meter (or a mobile terminal) exemplified in FIG. 1.
FIG. 3 is a block diagram illustrating a configuration example of a personal computer (PC) (or a server) exemplified in FIG. 1.
FIG. 4 is a flowchart for describing an operation example of an information processing system (an activity amount processing device) according to an embodiment.
FIG. 5 is a graph illustrating an example of activity amount data measured by an activity amount meter.
FIG. 6 is a graph illustrating an example in which calculated is a moving average with respect to the activity amount data exemplified in FIG. 5.
FIG. 7 is a graph illustrating an example of a result of frequency analysis performed on activity amount data of the first day of a week (seven days).
FIG. 8 is a graph illustrating an example of a result of frequency analysis performed on activity amount data of the second day of a week (seven days).
FIG. 9 is a graph illustrating an example of a result of frequency analysis performed on activity amount data of the third day of a week (seven days).
FIG. 10 is a graph illustrating an example of a result of frequency analysis performed on activity amount data of the fourth day of a week (seven days).
FIG. 11 is a graph illustrating an example of a result of frequency analysis performed on activity amount data of the fifth day of a week (seven days).
FIG. 12 is a graph illustrating an example of a result of frequency analysis performed on activity amount data of the sixth day of a week (seven days).
FIG. 13 is a graph illustrating an example of a result of frequency analysis performed on activity amount data of the seventh day of a week (seven days).
FIG. 14 is a table illustrating an example of a biorhythm (frequency f) of a test subject for each day obtained from the activity amount data of seven days exemplified in FIGs. 7 to 13, a time that is calculated from the biorhythm, and a time difference with respect to 24 hours.
FIG. 15 is a graph illustrating time waveforms of biorhythms of a test subject along with a time waveform of a circadian rhythm to be a standard.
FIG. 16 is a diagram illustrating a visualization example of information corresponding to a biorhythm according to an embodiment.
FIG. 17 is a diagram illustrating a visualization example of information corresponding to a biorhythm according to an embodiment.
FIG. 18 is a diagram illustrating a visualization example of information corresponding to a biorhythm according to an embodiment.
FIG. 19 is a diagram illustrating a visualization example of information corresponding to a biorhythm according to an embodiment.
FIG. 20 is a diagram illustrating a visualization example of information corresponding to a biorhythm according to an embodiment.
FIGs. 21A to 21D are diagrams each illustrating a visualization example of information corresponding to a biorhythm according to an embodiment. Description of Embodiments

A biorhythm is a periodic rhythm that a biological body autonomously beats, and in a case of a person, physiological fluctuations of sleep, body temperature, blood pressure, autonomic nerves, and the like are adjusted taking about 24 hours as one cycle. The biorhythm of a person is also referred to as a "circadian rhythm", "biological clock", or the like.

The "circadian rhythm" is usually synchronized with a cycle of light and dark of morning and night (about 24 hours). A case in which the "circadian rhythm" is disordered and is not synchronized with a cycle of about 24 hours (may also be referred to as "standard rhythm") is considered likely to affect the symptoms of jet lag, sleep disorder, seasonal depression, and the like. Therefore, treatment for improving the circadian rhythm is attracting attention.

A case in which the "circadian rhythm" of a test subject is shifted from the standard rhythm is considered likely to affect the exercise of physical performance of the test subject and the efficacy of medicines; for example, in the field of chronopharmacology, to maximize the efficacy or the like by normalizing the circadian rhythm is presently attracting attention. Accordingly, a technique capable of detecting the circadian rhythm of a test subject with ease is expected to be achieved.

For example, in a case where it is attempted to continuously measure the hair-root cells, blood, rectal temperature, or the like of a test subject for more than 24 hours in order to detect a circadian rhythm, the attempt discussed above will interfere with everyday life of the test subject. Accordingly, it may be difficult to apply such measurement in everyday life.

In addition, the test subject sleeps with a hormone (for example, melatonin) having influence on sleep being secreted by the circadian rhythm, but it is difficult to directly know the secretion of the hormone by, for example, a blood test.

Therefore, in the present embodiment, an attempt is made to detect periodicity derived from a biorhythm of a test subject based on data indicating an amount of activity including an activity state and a sleeping state of everyday life of the test subject. The "amount of activity" of the test subject may be rephrased as an "amount of movement" of the test subject. Further, the data indicating the amount of activity of the test subject may be shortly referred to as "activity amount data". The activity amount data may be time-series data capable of estimating vital information, a temporal transition of the activity state, and the like of the test subject.

In order to obtain, from the activity amount data, periodicity derived from the biorhythm, it is desirable that disturbance-induced factors different from physiological fluctuations of the test subject (for example, movement following physical activities of the test subject) be separated from the activity amount data.

If the above separation is allowed to be carried out, it is possible to find temporal "waves" of the amount of activity in a time period of about 24 hours, for example, it is possible to find the periodicity derived from the biorhythm.

Therefore, the inventors of the present application have found that it is possible to detect a biorhythm of a test subject with ease by detecting a component that represents one cycle in a time period of about 24 hours in a time waveform of activity amount data and that is unlikely to be affected by the movement following the physical activities of the test subject, for example. Note that "time waveform of activity amount data" may, in short, be referred to as "time waveform of activity amount".

Hereinafter, exemplary embodiments will be described with reference to the drawings. However, the embodiments described below are merely examples and are not intended to exclude an application of various variations or techniques which are not explicitly described below. Further, various exemplary aspects described below may be appropriately combined and carried out. Elements or components assigned the same reference numeral in the drawings used for the following embodiments will represent identical or similar elements or components unless otherwise specified.

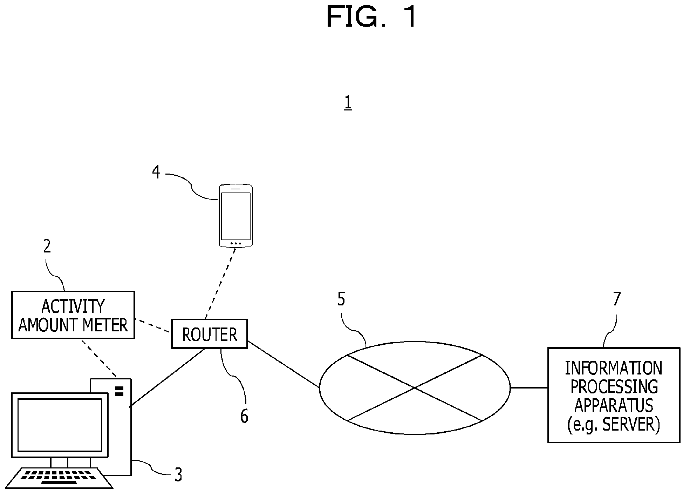

FIG. 1 is a block diagram illustrating a configuration example of an information processing system according to an embodiment. An information processing system 1 illustrated in FIG. 1 may illustratively include an activity amount meter 2, a personal computer (PC) 3, a mobile terminal 4, a network 5, a router 6, and an information processing apparatus 7.

The activity amount meter 2 is also called "life coder 2", and illustratively measures data indicating an amount of activity corresponding to the movement of a human body as an example of a biological body (this data may also be referred to as "activity amount data"). The activity amount meter 2 may be a contact type or may be a non-contact type. The activity amount meter 2 may be a wearable terminal. A non-limiting example of the activity amount meter 2 is a pedometer using an inertial sensor.

The activity amount meter 2 may be a specialized device for measuring the amount of activity, or may be a general-purpose or multi-functional device having an additional function different from that of measuring the amount of activity. A "person" as a measurement target by the activity amount meter 2 may also be called a "user", "observation target", or "test subject".

The number of test subjects may be plural, and an amount of activity of each of the plurality of test subjects may be measured by an individual activity amount meter 2. Alternatively, the activity amount meter 2 may be shared by part of or all of the plurality of test subjects. In a case where a single activity amount meter 2 is shared by the plurality of test subjects, the activity amount meter 2 may separately store the measurement results in a memory or the like for each of the plurality of test subjects.

Activity amount data obtained by the activity amount meter 2 may appropriately be inputted to the PC 3. The activity amount meter 2 may illustratively communicate with the PC 3 and input the activity amount data to the PC 3. The PC may be a desktop PC, a notebook PC (or laptop PC), a tablet PC, or the like.

The connection between the activity amount meter 2 and the PC 3 may be a wired connection or may be a wireless connection. Further, the communication between the activity amount meter 2 and the PC 3 may be communication through the router 6, or may be direct communication not through the router 6.

For the wired connection, as a non-limiting example, an applicable communication cable such as a local area network (LAN) cable or a Universal Serial Bus (USB) cable may be used. For the wireless connection, an applicable wireless communication system such as "Wireless Fidelity (WiFi)" (registered trademark), "Bluetooth" (registered trademark), or Near Field Communication (NFC) may be used.

The PC 3 may be communicably connected to the router 6, and may be capable of communicating with the information processing apparatus 7 via the network 5. Note that the "PC" is also an example of an "information processing apparatus".

The connection between the PC 3 and the router 6 may be a wired connection or may be a wireless connection. For the wired connection, as a non-limiting example, an applicable communication cable such as a LAN cable or a USB cable may be used. For the wireless connection, as a non-limiting example, an applicable wireless communication system such as "WiFi" or "Bluetooth" may be used.

The network 5 may illustratively be the Internet, a LAN, a wide area network (WAN), or the like. Further, the network 5 may include a wireless access network. The wireless access network may illustratively be a wireless access network based on the Long Term Evolution (LTE), the LTE-Advanced, or the like of the 3rd Generation Partnership Project (3GPP).

The information processing apparatus 7 may be a PC, or may be a server computer (may also be referred to as "server"). The server may be a cloud server installed in a data center or the like. Hereinafter, for the sake of convenience, it is assumed that the information processing apparatus 7 is a "server". The server 7 may receive and acquire the activity amount data by communicating with the PC 3 via the network 5.

In a case where the activity amount meter 2 is communicably connected to the router 6 being connected with the network 5, the server 7 may receive and acquire the activity amount data from the activity amount meter 2 via the network 5, not through the PC 3.

The connection between the activity amount meter 2 and the router 6 may be a wired connection or may be a wireless connection. For the wired connection, an applicable communication cable such as a LAN cable or a USB cable may be used. For the wireless connection, an applicable wireless communication system such as "WiFi", "Bluetooth", or "NFC" may be used.

Alternatively, the server 7 may receive and acquire, not through the router 6 but through the wireless access network, the activity amount data obtained by the activity amount meter 2.

The activity amount data may be measured by the mobile terminal 4. For example, the mobile terminal 4 may be provided with a function equivalent to part of or all of the function of the activity amount meter 2. The mobile terminal 4 may be a cellular phone such as a smartphone, or may be a wearable terminal.

The activity amount data obtained by the mobile terminal 4 may be received and acquired by the PC 3, the server 7, or the like through a communication path similar to that for the activity amount data obtained by the activity amount meter 2.

The activity amount data supplied from the activity amount meter 2, the mobile terminal 4, or the like to the PC 3, the server 7, or the like may be such data that all or part of the data has been processed or manipulated in the activity amount meter 2, the mobile terminal 4, or the like.

The PC 3, the server 7, or the like having acquired the activity amount data processes the acquired activity amount data. The processing of the activity amount data may include storing and managing the activity amount data. The managing of the activity amount data may include compiling the activity amount data in a database (DB). The data compiled in the DB may be referred to as "cloud data", "big data", or the like.

In addition, to "process the activity amount data" by the PC 3, the server 7, or the like may illustratively include a process of detecting a biorhythm of the test subject based on the acquired activity amount data. The "detection" of the biorhythm may also be referred to as "measurement", "determination", or "estimation".

Accordingly, the PC 3, the server 7, or the like may also be referred to as an activity amount processing device, or may be referred to as a biorhythm detection device, biorhythm measurement device, biorhythm determination device, or biorhythm estimation device as well. Hereinafter, for the sake of convenience, the PC 3, the server 7, or the like will be referred to as "activity amount processing device".

The function, processing (or algorithm), and the like as the activity amount processing device may be achieved by a single PC, a single server or the like, or may be achieved by the distributed processing configured of a plurality of PCs, servers, and the like.

In other words, the activity amount data, the biorhythm detection processing based on the activity amount data, or the like may be processed or managed by a single information processing apparatus, or may be processed or managed by a plurality of information processing apparatuses in a distributed manner.

Further, to "process the activity amount data" by the PC 3, the server 7, or the like may include a process to detect whether or not the test subject is sleeping (for convenience, it may be referred to as "sleeping state") based on the activity amount data acquired in time series in a certain time zone. The "detection" of the sleeping state may be rephrased as "measurement", "determination" or "estimation" of the sleeping state.

Whether the test subject is sleeping or not may be illustratively estimated by an arithmetic expression (may also referred to as "determination expression") called "AW2 expression" or "Cole expression".

For example, the PC 3 and the server 7 may determine that, in a case where a value calculated by the "AW 2 expression" or "Cole expression" based on the activity amount data having been acquired for a certain determination time (illustratively, several minutes) is equal to or greater than a certain threshold, the test subject is in a state of "sleep onset", and may determine that, in a case where the calculated value is smaller than the threshold, the test subject is in a state of "awakening".

Note that, the action of the test subject going to bed is referred to as "sleeping", and the action of the test subject awakening and leaving the bed is referred to as "waking up" or "leaving the bed" in some cases. "Sleeping" and "waking up" may be detected based on the magnitude (strength or power) of the amount of activity indicated by the activity amount data. The "magnitude of the amount of activity" may be referred to as "activity strength".

As a non-limiting example, "sleeping" may be detected by a situation where the activity strength less than a certain threshold has continued for a certain determination time (illustratively, several minutes). "Waking up" may be detected, as a non-limiting example, by a situation where the activity strength exceeding a certain threshold has continued for a certain determination time (illustratively, several minutes).

The thresholds and determination times used for detecting "sleeping" and "waking up" may be the same or may be different between the detection of "sleeping" and the detection of "leaving the bed". It is sufficient that the thresholds and determination times suitable for the detection of "sleeping" and "waking up" are set.

Further, to "process the activity amount data" by the PC 3, the server 7, or the like may illustratively include a process to visualize information on the biorhythm detected based on the activity amount data, information on comments, advice, or the like in accordance with the detected biorhythm, and the like.

For example, the PC 3, the server 7, or the like may visualize information indicating whether or not the biorhythm detected based on the activity amount data of the individual test subject is normal, information on an action recommended to be taken with respect to the detected biorhythm, and the like.

In other words, the information to be visualized may change in accordance with the biorhythm of the individual test subject that is detected based on the activity amount data. The information on the action recommended to be taken with respect to the biorhythm, may be information indicating how the biorhythm will be adjusted by what type of action is taken by the test subject with respect to the detected biorhythm.

For example, the information on the action recommended to be taken with respect to the biorhythm may be information on the action to be taken by the test subject capable of reducing a shift between the biorhythm and the standard rhythm. Since the biorhythm changes synchronously with the cycle of light and dark of the ambient environment, it is possible to adjust the biorhythm by controlling the time during which the test subject is exposed to light, the time during which the test subject is not exposed to light, or the like.

Therefore, for example, by controlling the sleeping time, the wake-up time, and the like of the test subject, it is possible to reduce the shift between the biorhythm of the test subject and the standard rhythm so as for the biorhythm to approach the normal state.

The "process to visualize" information by the PC 3, the server 7, or the like may illustratively include display processing (or display control) on a display, printing processing (or printing control) on a printer, and the like. The display may include a touch panel, a projector, or the like.

The display on which the information is displayed may be any of a display of the PC 3, a display of the server 7, a display of the activity amount meter 2, and a display of the mobile terminal 4. The printer by which the information is printed may be any of a printer coupled to the PC 3, a printer coupled to the server 7, a printer coupled to the activity amount meter 2, and a printer coupled to the mobile terminal 4.

### (Configuration Example of Activity Amount Meter 2)

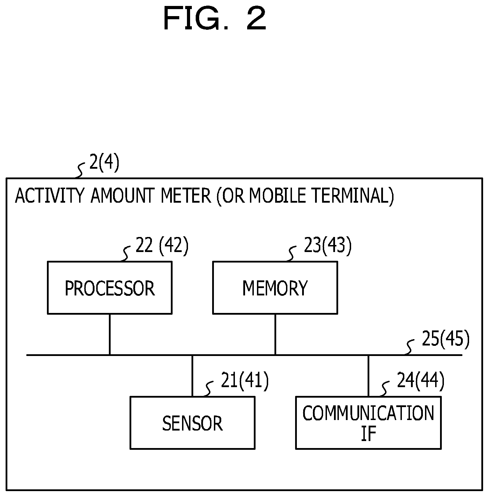

FIG. 2 illustrates a configuration example of the activity amount meter 2. Also, the mobile terminal 4 capable of measuring the activity amount data may have the configuration exemplified in FIG. 2, and elements of the mobile terminal 4 are indicated by reference numerals in parentheses.

Hereinafter, in order to avoid redundant description, elements 21 to 25 of the activity amount meter 2 will be described. In FIG. 2, the elements of the mobile terminal 4 indicated by reference numerals 41 to 45 in parentheses may be considered to have equivalent functions to those of the elements 21 to 25 of the activity amount meter 2, respectively, unless otherwise specified.

As illustrated in FIG. 2, the activity amount meter 2 may illustratively include an activity amount sensor 21, a processor 22, a memory 23, and a communication interface (IF) 24. The activity amount sensor 21, the processor 22, the memory 23, and the communication IF 24 may illustratively be coupled to a bus 25, and may be capable of communicating with each other through the processor 22.

It is sufficient that the activity amount sensor 21 (hereinafter, referred to as "sensor 21" in some cases) is illustratively a sensor which is capable of sensing the activity amount data corresponding to the activity of a biological body. Note that the activity amount data is an example of vital information. The activity amount data sensed by the sensor 21 may be referred to as "sensor data" for the sake of convenience. The term of "sensing" may be referred to as "detection" or "measurement".

As a non-limiting example, an inertial sensor, a radio wave sensor, a heartbeat sensor, a pulse sensor, or the like may be applied to the sensor 21 capable of measuring the activity amount data of a biological body.

The inertial sensor may be an acceleration sensor or a gyroscope. Any one of piezoelectric type and capacitive type sensors may be illustratively applied to the acceleration sensor. Any one sensor of a spin rotor (flywheel) type, an optical type, and a vibrating structure type may be applied to the gyroscope.

The inertial sensor may include one or more detection axes. A gravity component in a direction along the detection axis may be detected as "acceleration", for example. The detection of "acceleration" makes it possible to detect activity amount data in response to movement, posture, or the like of the biological body.

The activity amount meter 2 using an inertial sensor may be attached to bedding such as a bed. For example, the activity amount data of the test subject may be measured by detecting vibrations transmitted through the bedding due to the beating of the test subject's heart or the like with the inertial sensor attached to the bedding.

It is possible for the radio wave sensor to detect the movement of the biological body in a non-contact manner in which the radio wave sensor radiates radio waves such as microwaves to a sensing target, receives reflective waves, which are reflected at the sensing target, and detects the movement of the target based on a change of the reflective waves.

For example, in a case where a distance between the radio wave sensor and the sensing target is changed, a change occurs in the reflective waves due to the Doppler effect. The change of the reflective waves may be illustratively acquired as a change in one or both of amplitude and a frequency of the reflective waves. The "radio wave sensor" may also be referred to as a "microwave sensor", "radio frequency (RF) sensor", "ultra wide band (UWB) sensor" or "Doppler sensor".

The heartbeat sensor illustratively detects the pulse-beat of a blood vessel in response to the heartbeat of the biological body. For example, the heartbeat of the biological body may be acquired as a change of electromagnetic waves, pressure, or sound in response to the beating of the heart.

Illustratively, when a blood vessel of a finger, an earlobe or the like is irradiated with light such as infrared rays, the reflective light changes periodically in accordance with a rhythmical change of the bloodstream and the light absorption characteristics. Hence, it is possible to optically measure the heartbeat as a fluctuation of the reflective light in accordance with the bloodstream change.

Alternatively, when a biological body is irradiated with radio waves such as microwaves, a rhythmical motion occurs in a surface of the biological body (for example, skin) in accordance with beating of the heart, whereby a distance between the skin and a radio wave transmission source changes in accordance with the motion so that a change occurs in the reflective waves due to the Doppler effect.

Hence, it is also possible to measure the heartbeat of the biological body as a fluctuation in reflection, due to the Doppler effect, of the waves radiated on the biological body. To rephrase, the radio wave sensor may be used for the heartbeat sensor.

Further, when the heart is contracted and relaxed rhythmically, the pressure of a blood vessel (hereinafter, may be referred to as "blood pressure") is also fluctuated rhythmically, whereby it is also possible to measure the heartbeat as a rhythmical fluctuation of the blood pressure by using a pressure sensor, a piezoelectric sensor, or the like.

Furthermore, it is also possible to measure the heartbeat as a change of electric potential or a change of sound of the heart muscle corresponding to the heart beating like in a case of using an electrocardiograph or a phonocardiograph.

As described above, since the information indicative of the heartbeat may be considered as being equivalent to the information indicative of the pulse in some cases, the "heartbeat sensor" may be referred to as a "pulse sensor".

The activity amount meter 2 including the sensor 21 may also be referred to as "sensor unit 2" for the sake of convenience. The sensor unit 2 may illustratively be mounted in contact with the skin of a human body, or mounted at a position separate from the human body in a non-contact state within a range where it is possible to sense the vital information.

The activity amount data supplied from the activity amount meter 2, the mobile terminal 4, or the like to the PC 3, the server 7, or the like may be primary data detected by the sensor 21 or may be secondary data obtained based on the detected data. To rephrase, it is sufficient for the activity amount data to be such data that represents the movement corresponding to the activity of everyday life of the test subject.

The processor 22 is an example of an arithmetic processing unit having the capability of arithmetic processing. The arithmetic processing unit may be referred to as an arithmetic processing device or an arithmetic processing circuit. A central processing unit (CPU), a digital signal processor (DSP), a micro processing unit (MPU), an integrated circuit (IC), or the like may be illustratively applied to the arithmetic processing unit. The "processor" may be referred to as a "processing unit", a "controller" or a "computer".

The processor 22 illustratively implements a function, processing, and the like as the activity amount meter 2, and controls operations corresponding to the above function, processing, and the like. A program, data, and the like for implementing the function, processing, control, and the like as the activity amount meter 2, may be stored in the memory 23.

The "program" may be referred to as "software" or an "application". The "data" may include the activity amount data and the data generated according to the operations of the processor 22.

The processor 22 is configured to operate while reading out the program, data, and the like stored in the memory 23, by which the function, processing, and control are implemented as the activity amount meter 2. The memory 23 is an example of a storage medium, and may be a random access memory (RAM), a flash memory, or the like.

All or part of program codes configuring a program may be stored in a storage section or may be described as part of an operating system (OS).

The program and the data may be recorded in a computer-readable recording medium to be provided. Examples of the recording medium include a flexible disk, CD-ROM, CD-R, CD-RW, MO, DVD, Blu-ray Disk, portable hard disk, and the like. Further, a semiconductor memory such as a Universal Serial Bus (USB) memory is also an example of the recording medium.

Alternatively, the program and the data may be provided (for example, downloaded) from the server or the like to the activity amount meter 2 via the network 5. For example, the program and the data may be provided to the activity amount meter 2 through the communication IF 24.

The communication IF 24 is an example of a communication unit included in the activity amount meter 2, and illustratively makes it possible to communicate with the PC 3, the router 6, and the wireless access network. The activity amount data obtained by the sensor 21 or the processor 22 may be transmitted to the PC 3, the server 7, and the like through the communication IF 24.

### (Configuration Example of PC 3)

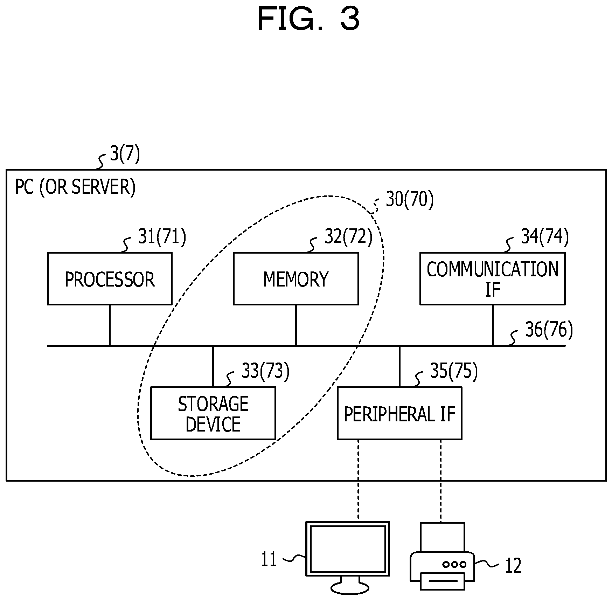

Next, FIG. 3 illustrates a configuration example of the PC 3. Since the PC 3 is an example of an information processing apparatus, the configuration thereof may be similar to that of the server 7. Therefore, elements of the server 7 are indicated by reference numerals 71 to 76 in parentheses in FIG. 3.

Hereinafter, in order to avoid redundant description, elements 31 to 36 of the PC 3 will be described. In FIG. 3, the elements of the server 7 indicated by the reference numerals 71 to 76 in parentheses may be considered to have equivalent functions to those of the elements 31 to 36 of the PC 3, respectively, unless otherwise specified.

As illustrated in FIG. 3, the PC 3 may illustratively include a processor 31, a memory 32, a storage unit 33, a communication IF 34, and a peripheral IF 35. The processor 31, the memory 32, the storage unit 33, the communication IF 34, and the peripheral IF 35 may be illustratively coupled to a bus 36, and may be capable of communicating with each other through the processor 31.

The processor 31 is an example of an arithmetic processing unit having the capability of arithmetic processing. The arithmetic processing unit may be referred to as an arithmetic processing device or an arithmetic processing circuit. For example, a CPU, a DSP, an MPU, an IC, or the like may be applied to the arithmetic processing unit. The "processor" may be referred to as a "processing unit", a "controller" or a "computer".

The processor 31 illustratively causes the PC 3 to function or carry out processing as an activity amount processing device, and controls the operations corresponding to the function and the processing. A program and data for implementing the function, processing, and control as the activity amount processing device 3, may be stored in the memory 32 and the storage unit 33.

The processor 31 is configured to operate while reading out the program and the data stored in the memory 32 and the storage unit 33, by which the function, processing, and control are implemented as the activity amount processing device 3.

The memory 32 is an example of a storage medium, and may be a RAM, a flash memory, or the like.

The storage unit 33 may store various types of data and programs. A hard disk drive (HDD), a solid state drive (SSD), a flash memory, or the like may be used for the storage unit 33.

The data stored in the storage unit 33 may illustratively include the activity amount data received through the communication IF 34. The data stored in the storage unit 33 may be appropriately compiled in a database (DB). The data compiled in the DB may be referred to as "cloud data", "big data", or the like.

The storage unit 33 and the memory 32 may be collectively referred to as a "storage section 30" of the PC 3. Likewise, the storage unit 73 and the memory 72 in the server 7 may also be collectively referred to as a "storage section 70".

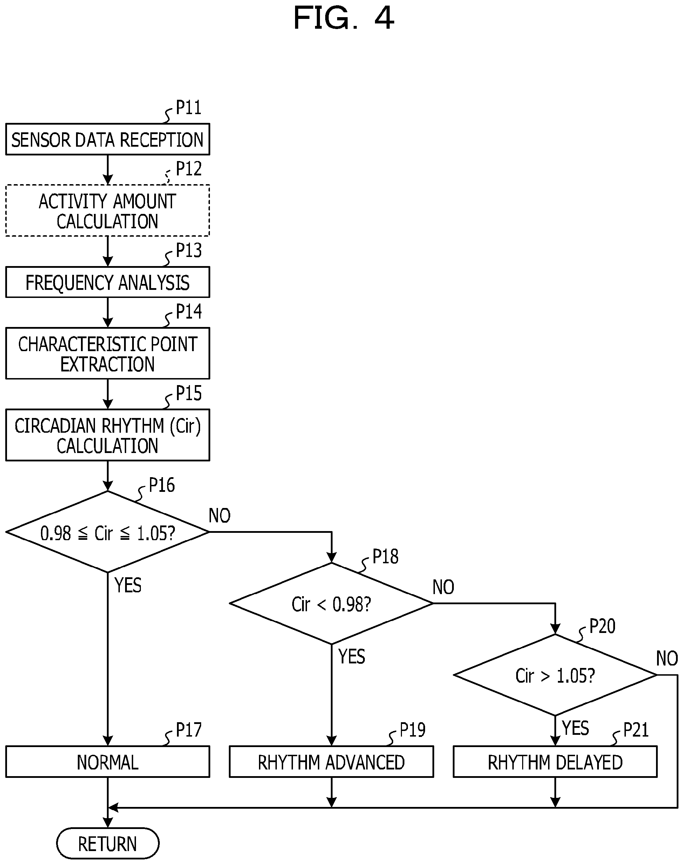

The programs stored in the storage section 30 may include a program to carry out processing, which will be described later with reference to FIG. 4. This program may be referred to as an "activity amount processing program" for the sake of convenience.

All or part of program codes configuring a program may be stored in the storage section or may be described as part of an OS.

The program and the data may be recorded in a computer-readable recording medium to be provided. Examples of the recording medium include a flexible disk, CD-ROM, CD-R, CD-RW, MO, DVD, Blu-ray Disk, portable hard disk, and the like. Further, a semiconductor memory such as a Universal Serial Bus (USB) memory is also an example of the recording medium.

Alternatively, the program and the data may be provided (for example, downloaded) from the server or the like to the PC 3 via the network 5. For example, the program and the data may be provided to the PC 3 through the communication IF 34.

The communication IF 34 is an example of a communication unit included in the PC 3, and illustratively makes it possible to communicate with the activity amount meter 2, the mobile terminal 4, the router 6, the wireless access network, and the like.

Focusing on reception processing, the communication IF 34 is an example of a receiver (which may be referred to as an "acquisition unit") configured to receive the activity amount data obtained by the activity amount meter 2 or the mobile terminal 4. On the other hand, focusing on transmission processing, the communication IF 34 is an example of a transmitter configured to transmit the activity amount data obtained by the activity amount meter 2 or the mobile terminal 4 to the server 7, for example.

A communication IF 74 of the server 7 is illustratively capable of transmitting data to the activity amount meter 2, the PC 3, and the mobile terminal 4 via the network 5. The data transmitted to the activity amount meter 2, the PC 3, and the mobile terminal 4 may illustratively include a processed result of activity amount data (for example, a determination result of a circadian rhythm).

The peripheral IF 35 is illustratively an interface for coupling peripheral devices to the PC 3. The peripheral devices may include input devices configured to input information to the PC 3, and output devices configured to output the information generated by the PC 3.

The input devices may include a keyboard, a mouse, a touch panel, and the like. The output devices may include a display 11, a printer 12, and the like, as schematically exemplified in FIG. 3.

### (Operation Example)

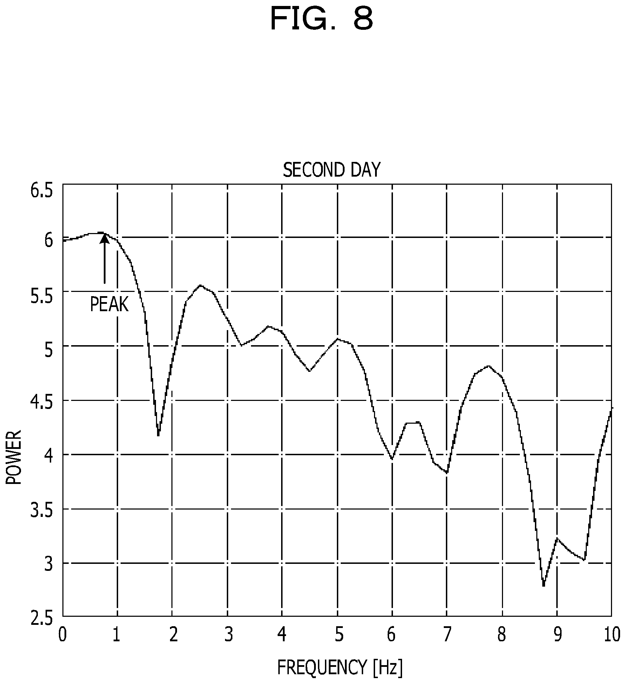

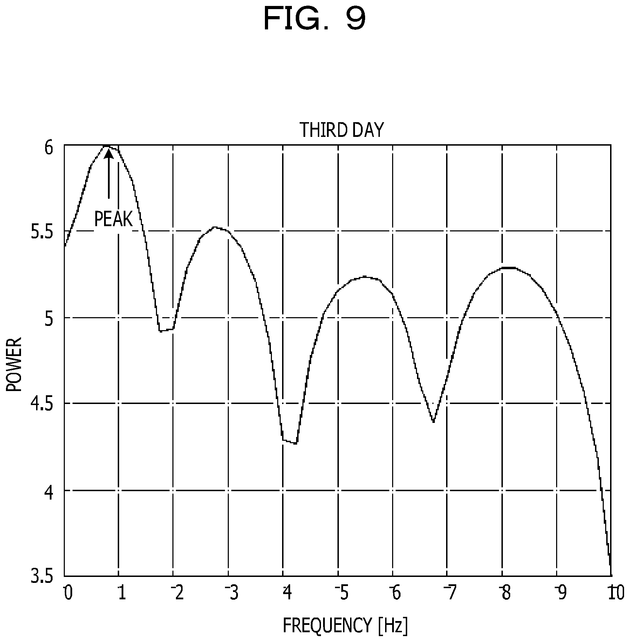

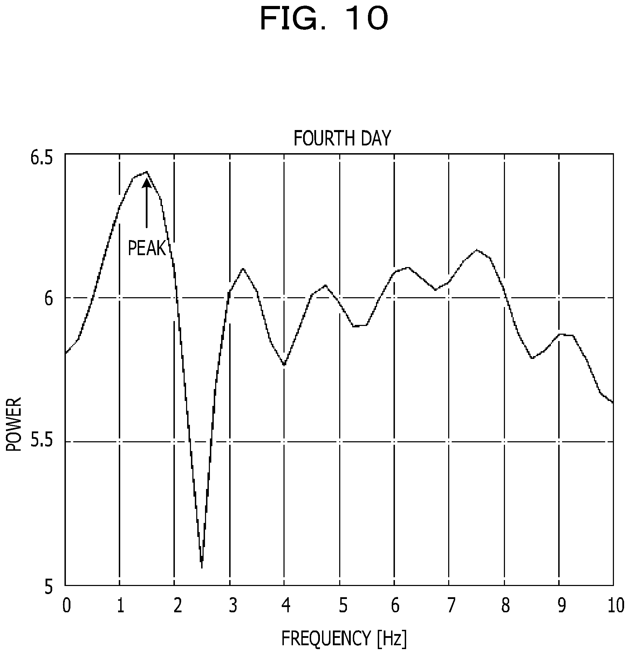

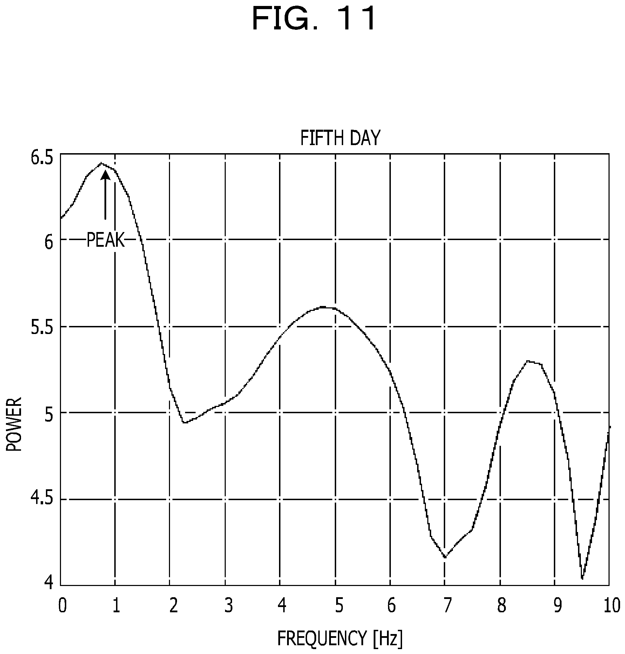

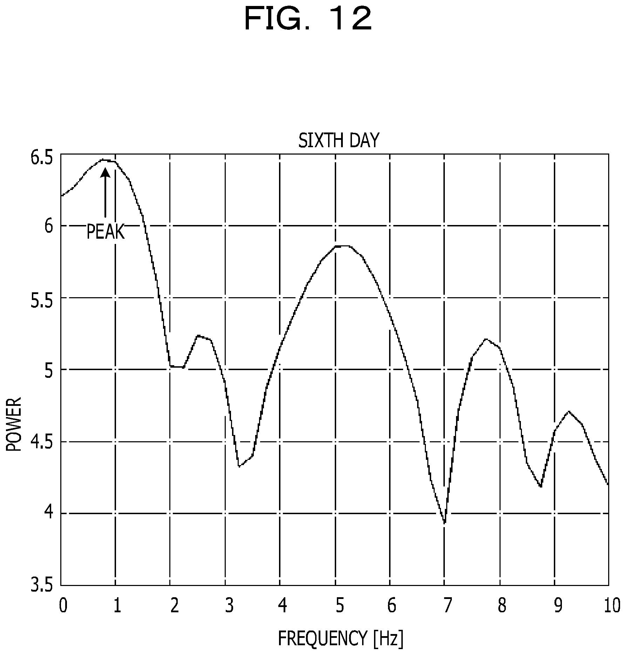

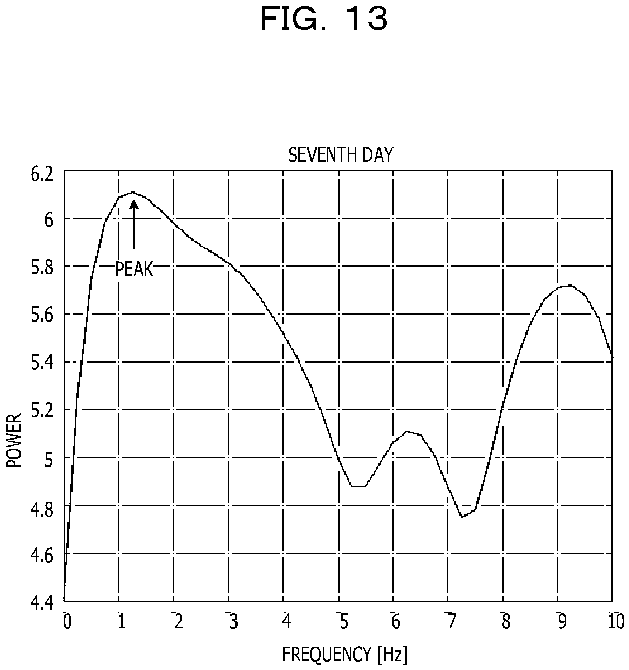

Hereinafter, an operation example of the information processing system 1 will be described with reference to FIGs. 4 to 20. Note that in the following description of the operation example, for the sake of convenience, an example in which the PC 3 operates as an "activity amount processing device" and carries out a detection process of a circadian rhythm based on activity amount data will be described. Further, it is assumed that the activity amount data processed by the activity amount processing device 3 is activity amount data having been measured by the activity amount meter 2.

Note that, all or part of the operation example described below may be replaced with an operation example of the server 7 (for example, a processor 71), which operates as an "activity amount processing device". Further, in the operation example described below, "the activity amount meter 2" may be replaced with "the mobile terminal 4".

FIG. 4 is a flowchart illustrating an operation example of the activity amount processing device 3. The flowchart exemplified in FIG. 4 may be illustratively executed by the processor 31 of the activity amount processing device 3.

As exemplified in FIG. 4, the activity amount processing device 3 receives sensor data measured by the activity amount meter 2 (processing P11).

The sensor data may be activity amount data, or may be data from which it is possible to calculate activity amount data. In the case of the latter one from which it is possible to calculate the activity amount data, the activity amount processing device 3 may calculate the activity amount data based on the above data (processing P12).

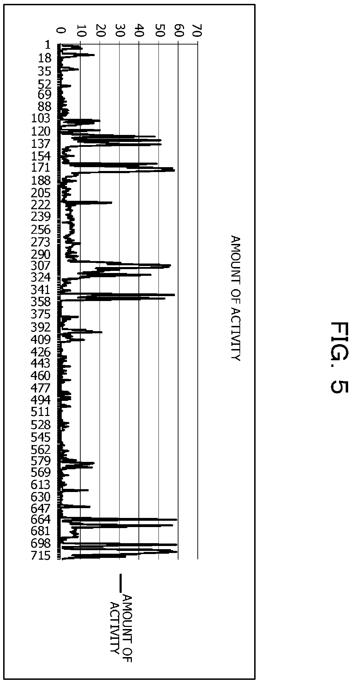

FIG. 5 illustrates an example of the activity amount data. In FIG. 5, as a non-limiting example, a change in activity amount of a test subject for one day (= 24 hours) is illustrated in the case of using an inertial sensor as the activity amount sensor 21 (or 41).

In FIG. 5, the horizontal axis represents time (illustratively represents sampling timings), and the vertical axis represents activity amount power. The horizontal axis of FIG. 5 indicates that, as a non-limiting example, activity amount data is sampled 720 times in 24 hours and 720 pieces of sampling data (may be referred to as "time-series data") are obtained. To rephrase, the sampling rate of the activity amount data is 720 Hz.

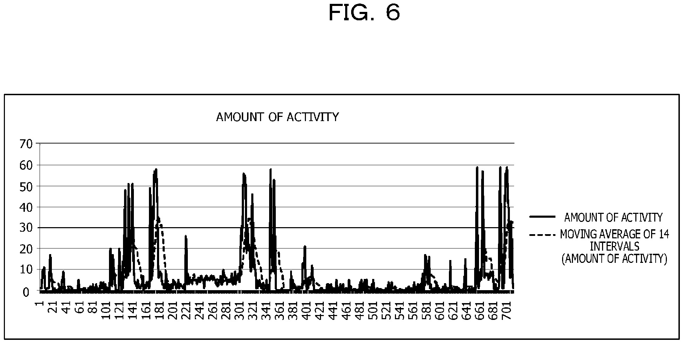

The activity amount processing device 3 detects, from the activity amount data as exemplified in FIG. 5, a circadian rhythm of the test subject (in other words, periodicity of approximately 24 to 25 hours). Here, in a case where the activity amount data of 24 hours is smoothed by calculating a moving average of, for example, 14 intervals with respect to the time-series activity amount data exemplified in FIG. 5, a certain periodicity appears as illustrated in FIG. 6, for example.

However, since the above periodicity depends on the magnitude of the original activity amount data (may be referred to as "raw data"), in other words, depends on the magnitude of the movement following the activity of the test subject, the stated periodicity is largely different from the period of the circadian rhythm. Therefore, it is difficult to detect a circadian rhythm even if the activity amount data is smoothed by the moving average.

Then, the activity amount processing device 3 (for example, the processor 31) of the present embodiment illustratively acquires the time-series activity amount data as a time waveform and performs frequency analysis on the time waveform (processing P13), and detects and extracts a characteristic point in the frequency analysis result (processing P14).

The fast Fourier transform (FFT) or the discrete Fourier transform (DFT) may be applied to the frequency analysis. The "characteristic point" is a characteristic frequency indicating the periodicity of the circadian rhythm. For example, the "characteristic point" may be a frequency component having a peak of power spectrum density (which may be referred to as "peak power") in the frequency analysis result.

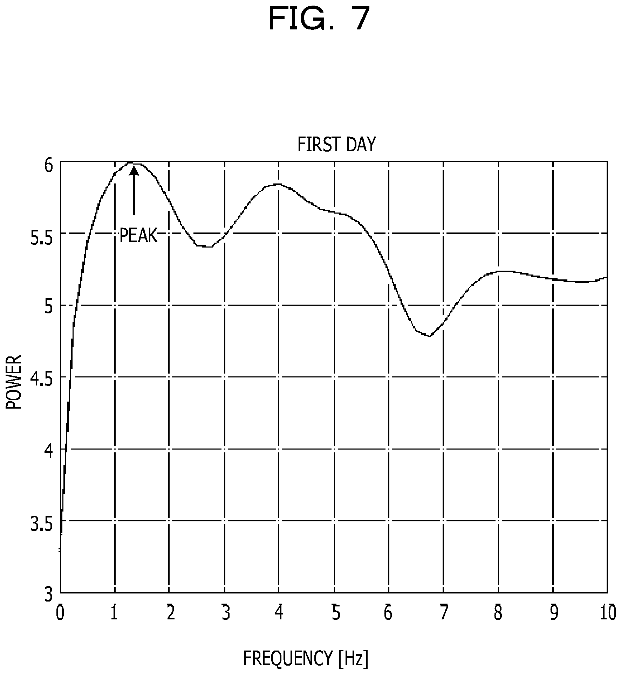

In FIGs. 7 to 13, examples of the results of frequency analysis having been performed on each activity amount data of one week (seven days) are illustrated. In each of FIGs. 7 to 13, the horizontal axis represents a frequency, and the vertical axis represents power. The activity amount processing device 3 may search for a frequency having peak power (see arrow marks) and may detect and extract the stated frequency as a "characteristic point", in each of the frequency analysis results exemplified in FIGs. 7 to 13.

As exemplified in FIGs. 7 to 13, the frequency having peak power (which may be referred to as "peak frequency" for the sake of convenience) is a frequency which completes one cycle near approximately 24 hours a day. In the case where 24 hours a day are illustratively defined as 1 Hz in a pseudo manner, the activity amount processing device 3 may search for a peak frequency closest to 1 Hz in the frequency analysis result.

The reason why 24 hours a day are defined as 1 Hz in the pseudo manner is as follows. For example, when a period is expressed as T [sec], a frequency f [Hz] of the wave is expressed by an equation of f = 1 / T [Hz]. Since 24 hours are equal to a value of 60 sec. x 60 x 24 = 86400 seconds, f becomes equal to 11.57 [µHz] in a case where 24 hours a day correspond to one wavelength.

Here, in the case where the frequency analysis is performed on the activity amount data, 86400 samples are obtained by the activity amount data of 24 hours being sampled every second; in other words, in the case where the sampling rate is 86400 Hz, the frequency to be determined is not balanced with respect to the number of samples. Due to this, the amount of calculation becomes large compared to the number of samples.

Then, a single day is assumed to be just one second in a pseudo manner, and it is defined that a one-day period is equivalent to 1 Hz. The activity amount processing device 3 finds a period of the peak frequency closest to 1 Hz in the frequency analysis result, so as to calculate a circadian rhythm as a biological rhythm (which may be referred to as "cycle") of one day of the test subject (processing P15 in FIG. 4).

The same result is obtained in a case of 86400 seconds being thinned out to be one hundred twentieth thereof, that is, 720 seconds, or in a case of being accumulated. Therefore, it is possible to significantly reduce the amount of calculation by carrying out the processing in which the number of samples is decreased from 86400 to 720, that is, the sampling rate is set to be 720 Hz.

Since a single day is defined as 1 Hz in a pseudo manner, a frequency lower than 1 Hz may be set as a search frequency in the result obtained through the frequency analysis of the activity amount data of a time period that is shorter than 24 hours such as 18 hours (3/4 days) or 12 hours (1/2 day).

For example, in the case of the frequency analysis result of the 3/4-day activity amount data, it is sufficient to search for a peak frequency closest to 0.75 Hz; in the case of the frequency analysis result of the 1/2-day activity amount data, it is sufficient to search for a peak frequency closest to 0.5 Hz.

To rephrase, the activity amount data or the frequency analysis result used for detecting a circadian rhythm may be data of a time period less than 24 hours.

For example, since the circadian rhythm of a test subject may be reset by the test subject being exposed to sunlight, activity amount data of several hours from when the test subject wakes up in the morning may not exhibit a characteristic periodicity of the circadian rhythm.

Accordingly, in a case where 24 hours a day are divided by, for example, six hours into 4 parts, activity amount data or a frequency analysis result corresponding to the activity amount data or the frequency analysis result of about six to twelve hours (1/4 day to 1/2 day) from the wake-up, may be excluded from candidates to be used in the calculation of a circadian rhythm. This makes it possible to reduce an amount of calculation processing of the circadian rhythm and suppress a decrease in calculation accuracy of the circadian rhythm.

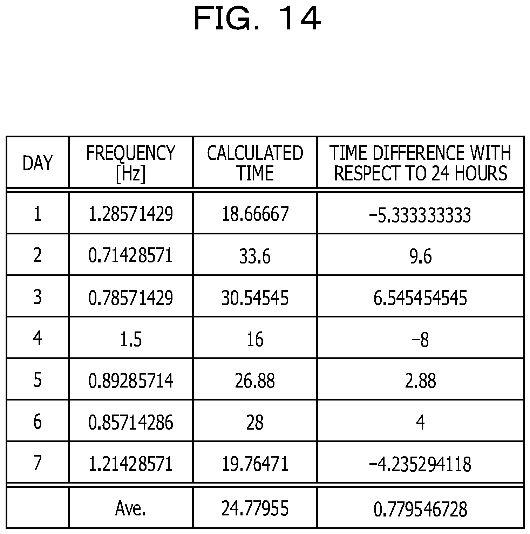

FIG. 14 illustrates an example of a biorhythm (frequency f) of a test subject for each day obtained from the activity amount data of seven days exemplified in FIGs. 7 to 13.

It is possible for a rhythm Cir for one hour unit with respect to the frequency f ("calculated time" in FIG. 14) to be expressed by an equation of Cir = 24/f. Thus, it is possible to obtain a time difference of the rhythm Cir with respect to 24 hours a day by "24 - Cir".

Accordingly, from the data exemplified in FIG. 14, it is possible to detect and determine the degree of shift of the biorhythm of the individual test subject from 24 hours a day.

For example, in FIG. 14, since each of the frequency f of the first, fourth, and seventh day is higher than 1 Hz, the time difference of the rhythm with respect to 24 hours takes a "negative" value.

Accordingly, it is possible to determine that the circadian rhythm of the test subject is shorter (reduced) than 24 hours to be the standard time. The circadian rhythm being shorter than 24 hours may cause early-morning awakening, for example.

On the other hand, in FIG. 14, since each of the frequency f of the second, third, fifth, and sixth day is lower than 1 Hz, the time difference of the rhythm with respect to 24 hours takes a "positive" value.

Accordingly, it is possible to determine that the circadian rhythm of the test subject is longer (extended) than 24 hours to be the standard time. The circadian rhythm being longer than 24 hours may bring about, for example, a symptom such as having difficulty in waking up or having difficulty in falling asleep at night.

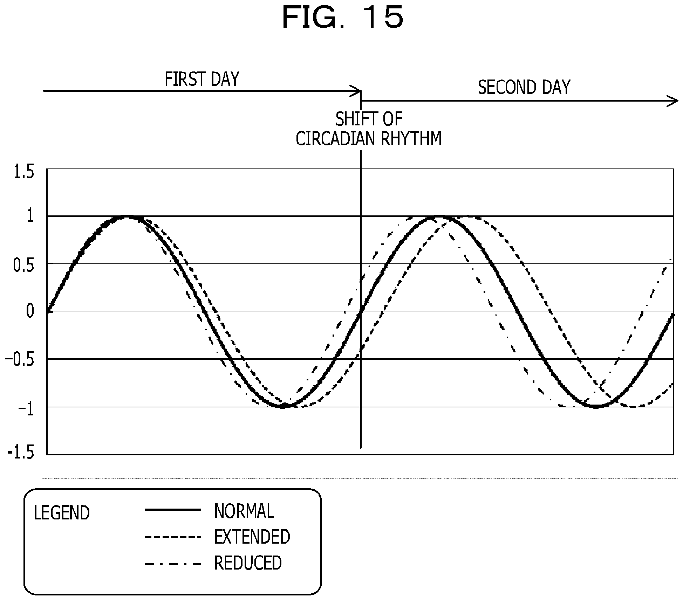

FIG. 15 illustrates an example of time waveforms of a rhythm Cir for two days of a test subject. In FIG. 15, a waveform depicted with a solid line represents a normal waveform in which the circadian rhythm of the test subject is close to 24 hours. In contrast, a waveform depicted with a dot-dash line represents a waveform of a case in which the circadian rhythm of the test subject is shorter than 24 hours, and a waveform depicted with a dotted line represents a waveform of a case in which the circadian rhythm of the test subject is longer than 24 hours.

The circadian rhythm beats a rhythm at a period of about 24 to 25 hours a day, and forms two wavelengths in two days in the case where the circadian rhythm is normal. In the case where the circadian rhythm becomes longer, since the circadian rhythm is less than two wavelengths, a body rhythm becomes slow so that a symptom such as having difficulty in waking up or having difficulty in falling asleep at night may be caused as discussed above. Conversely, in the case where the circadian rhythm becomes shorter, since the circadian rhythm exceeds two wavelengths in two days, the body rhythm becomes faster so that early-morning awakening may be caused as discussed above.

Examples of symptoms that may occur in a person when the circadian rhythm shifts from the normal rhythm are listed below.

### (1) Sleep phase delay syndrome

It is a symptom in which the sleeping time zone temporally shifts backward so that the patient falls asleep only late at night. The patient tries in vain to sleep earlier, and is likely to finally feel drowsy in a time zone from nighttime to dawn.

### (2) Advanced sleep phase syndrome

It is a symptom in which the sleeping time zone temporally shifts forward so that the patient becomes sleepy at an excessively early time. Strong drowsiness occurs in an early time zone of the night or occurs from the evening, and the awakening is likely to occur in the middle of the night or early in the morning. It is said to be a symptom more common in the elderly.

### (3) Non-24-hour sleep-wake syndrome

It is a symptom in which the bedtime and wake-up time are delayed by one to two hours every day. The biological clock does not work normally, and is not reset even in the morning.

### (4) Irregular sleep-wake pattern

The biological clock is in a completely broken state, so that the rhythm of sleep is lost and irregular drowsiness appears irrespective of day and night.

### (5) Shift work sleep disorder

It is a sleep disorder in which the circadian rhythm is disordered by continuing shift work, late-night work, or the like for a long time. A symptom occurs in which it takes long to fall asleep and the sleep is light during sleeping.

### (6) Jet lag syndrome

It is a symptom that a traveler may suffer in a case where the traveler moves to a region with a time difference of four to five hours or more at high speed by airplane or the like so that the biological clock is shifted from the cycle of light and dark of the external environment. However, it is a transient symptom in many cases.

The activity amount processing device 3 may determine whether or not the biorhythm (Cir) of the individual test subject falls within a range capable of being determined to be normal (may also be referred to as "normal range"), and may visualize the information in accordance with the determination result. The device on which the information is visualized may be the display 11 of the PC 3 or the server 7, the printer 12 or the like, or may be the display of the activity amount meter 2 or the mobile terminal 4.

The information according to the determination result of the biorhythm may be comments, advice, or the like regarding the lifestyle, sleep, or the like of the individual test subject.

In order to determine whether or not the biorhythm (Cir) of the individual test subject falls within the normal range, for example, as illustrated in FIG. 4, the activity amount processing device 3 may determine whether or not the biorhythm Cir obtained in the processing P15 falls within a range of 0.98 ≤ Cir ≤ 1.05 (processing P16). As Cir is closer to 1.0 (in other words, as the frequency f is closer to 1 Hz), it is indicated that the shift of the biorhythm from the standard rhythm is smaller.

In the case where the biorhythm Cir falls within the range of 0.98 ≤ Cir ≤ 1.05 (in the processing 16, YES), the activity amount processing device 3 may determine that the biorhythm Cir of the individual test subject is "normal".

In other words, "0.98" is an example of the lower limit value (first threshold) of Cir when the biorhythm of the test subject may be determined to be "normal", and "1.05" is an example of the upper limit value (second threshold) of Cir when the biorhythm of the test subject may be determined to be "normal".

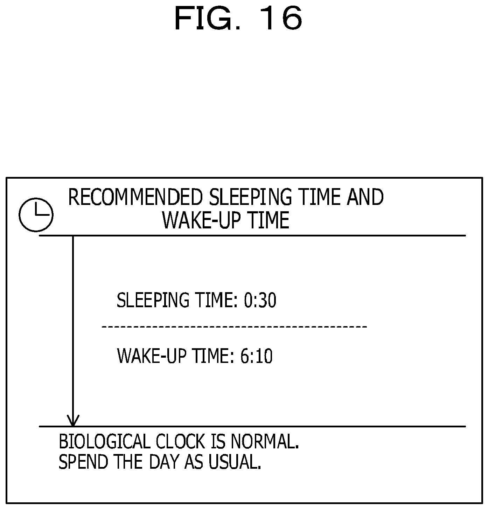

In response to the determination that the biorhythm Cir of the individual test subject is normal, the activity amount processing device 3 may visualize information indicating that the biorhythm Cir of the individual test subject is normal (processing P17), as schematically illustrated in FIG. 16, for example.

In addition, the activity amount processing device 3 may visualize, along with the information indicating that the biorhythm Cir of the individual test subject is normal, information indicating that it is not required to adjust a sleeping time (or may be a sleep onset time; the same is applied hereinafter) and a wake-up time, for example. The information indicating that it is not required to adjust the sleeping time and the wake-up time is an example of information indicating that it is not required to adjust the biorhythm.

Further, the activity amount processing device 3 may visualize, along with the information indicating that it is not required to adjust the sleeping time and the wake-up time, information of the sleeping time and the wake-up time of the test subject of the previous day, for example. This makes it easy for the test subject to visually recognize that it is sufficient to sleep and wake up at the same sleeping time and the same wake-up time as usual (for example, as the previous day).

On the other hand, in the case where the biorhythm Cir obtained in the processing P15 does not fall within a predetermined range, for example, a range of 0.98 ≤ Cir ≤ 1.05 (in the processing P16, NO), the activity amount processing device 3 may further determine whether or not the biorhythm Cir is less than 0 98 (Cir < 0.98) (processing P18).

In the case where the biorhythm Cir is less than 0.98, the activity amount processing device 3 may determine that the body rhythm of the test subject is earlier (more advanced) than the standard rhythm (in the processing P18, YES).

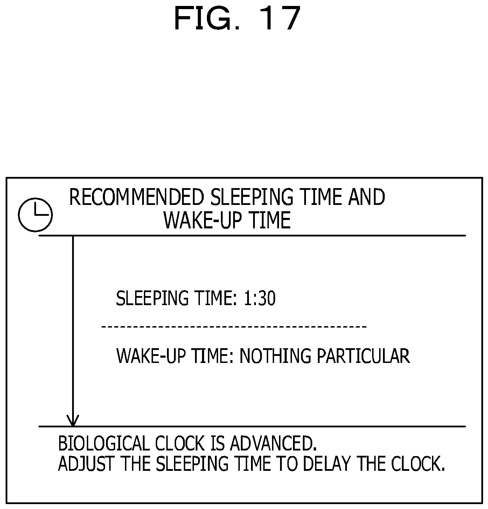

In response to the above determination, the activity amount processing device 3 may visualize, as schematically illustrated in FIG. 17, for example, information indicating that the body rhythm of the test subject is more advanced than the standard rhythm (for convenience, it may be referred to as "early arrival") (processing P19).

Here, in order to delay the biorhythm that is more advanced than the standard rhythm, it is considered effective to delay the sleeping time more than usual (for example, the previous day) or to be exposed to strong light in the evening.

Then, as schematically illustrated in FIG. 17, for example, the activity amount processing device 3 may visualize advice to make the sleeping time more delayed than usual because of the biorhythm being advanced. For example, the advice may be "Sleep after xx o'clock", or the like. The above advice is an example of information on the action of the test subject capable of delaying the biorhythm.

At that time, the visualization of the advice on the wake-up time may not be required. The reason for this is as follows: in the case where the biorhythm is advanced, even if the wake-up time is adjusted, the effect obtained by adjusting the wake-up time tends to be smaller than that obtained by adjusting the sleeping time. However, the advice on the wake-up time is not prohibited.

An adjustment time Td of the sleeping time to be recommended may be illustratively calculated by an equation of Td = (24 - (24 × Cir)) / 3 [hour]. The activity amount processing device 3 may obtain the sleeping time to be recommended to the test subject by adding the calculated adjustment time Td of the sleeping time to the sleep onset time of the test subject of the previous day.

For example, since the adjustment time Td is 1.04 when Cir is 0.87, in a case where the sleep onset time of the test subject of the previous day is 12:30 a.m., the activity amount processing device 3 may take 1:30 a.m., delayed by about one hour from 12:30 a.m., as the sleeping time recommended to the test subject.

On the other hand, in the case where the biorhythm Cir is not smaller than 0.98 (NO) in the processing P18 in FIG. 4, the activity amount processing device 3 may further determine whether or not the biorhythm Cir exceeds 1.05 (processing P20).

In the case where the biorhythm Cir exceeds 1.05 (in processing P20, YES), the activity amount processing device 3 may determine that the body rhythm of the test subject is behind the standard rhythm.

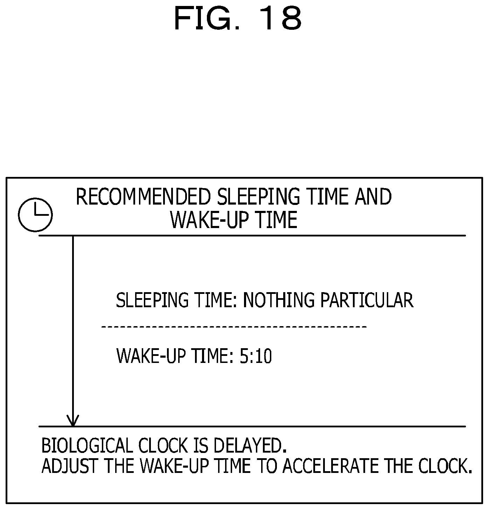

In response to this determination, the activity amount processing device 3 may visualize information indicating that the body rhythm of the test subject is behind the standard rhythm, as schematically illustrated in FIG. 18 (processing P21), for example.

Here, in order to accelerate the biorhythm that is behind the standard rhythm, it is considered effective to make the wake-up time earlier than usual or to be exposed to strong light in the morning.

Then, as schematically illustrated in FIG. 18, for example, the activity amount processing device 3 may visualize advice to make the wake-up time earlier than usual because of the biorhythm being delayed. The above advice is an example of information on the action of the test subject capable of making the biorhythm earlier.

At that time, the visualization of the advice on the sleeping time may not be required. This is because it may be easier for the test subject to follow the advice on the wake-up time than the advice on the sleeping time in some case, and there is a case in which a sufficient biorhythm adjustment effect is obtained by adjusting the wake-up time. However, the advice on the sleeping time is not prohibited.

An adjustment time Ta of the wake-up time to be recommended may be illustratively calculated by an equation of Ta = ((24 × Cir) - 24) / 3 [hour]. The activity amount processing device 3 may obtain the wake-up time to be recommended to the test subject by subtracting the calculated adjustment time Ta of the wake-up time from the wake-up time of the test subject of the previous day.

For example, since the adjustment time Ta is 1.04 when Cir is 1.13, in a case where the wake-up time of the test subject of the previous day is 6:10 a.m., the activity amount processing device 3 may take 5:10 a.m., earlier by about one hour from 6:10 a.m., as the wake-up time recommended to the test subject.

In the processing P20 in FIG. 4, in the case where the biorhythm Cir is not larger than 1.05 (NO), the activity amount processing device 3 may not visualize information on the biorhythm.

Not all of the processing P11 to P21 exemplified in FIG. 4 may be carried out in the PC 3, the server 7, or the like. Part of the processing P11 to P21 may be executed by the processor 22 of the activity amount meter 2 or a processor 42 of the mobile terminal 4 in accordance with the processing capacity of the processor 22 of the activity amount meter 2 or the processor 42 of the mobile terminal 4.

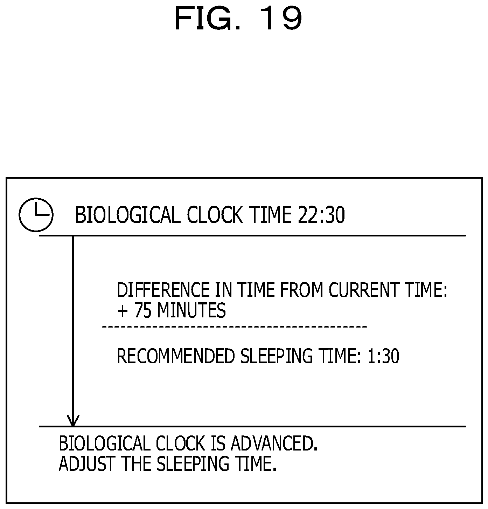

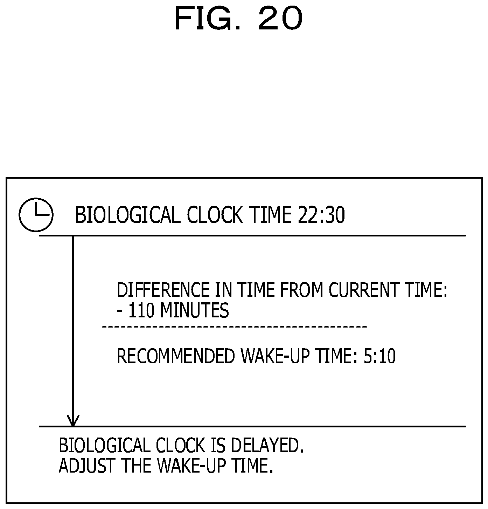

Further, as illustrated in FIGs. 19 and 20, for example, the activity amount processing device 3 may visualize information indicating a difference between the current time of the biological clock of the test subject obtained based on the cycle that the detected biorhythm beats and the actual current time in 24 hours a day.

In the mode of visualization illustrated in FIG. 19, it is visualized that the current time of the biological clock of the test subject is "22:30", and is advanced from the actual current time by a time difference of "+ 75 minutes".

Therefore, in this case, similarly to the example of FIG. 17, the activity amount processing device 3 may visualize information on the sleeping time recommended to the test subject as advice effective for delaying the biorhythm of the test subject.

On the other hand, in the mode of visualization illustrated in FIG. 20, it is visualized that the current time of the biological clock of the test subject is "20:30", and is delayed from the actual current time by a time difference of "- 110 minutes".

Therefore, in this case, similarly to the example of FIG. 18, the activity amount processing device 3 may visualize information on the wake-up time recommended to the test subject as advice effective for accelerating the biorhythm of the test subject.

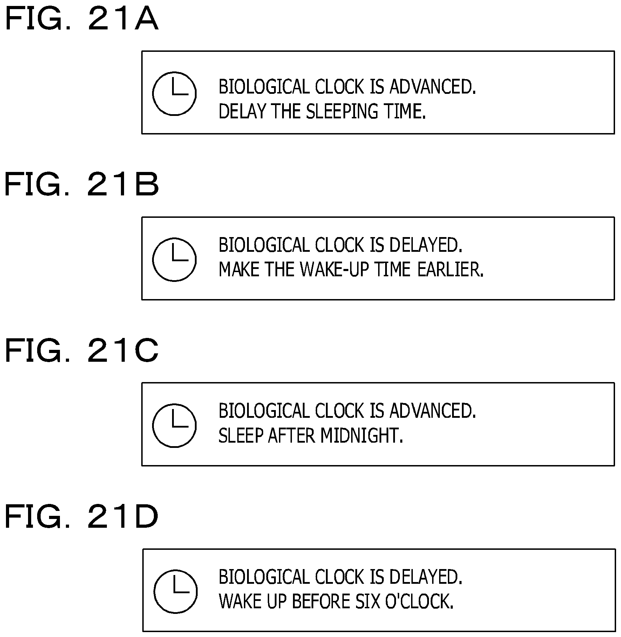

In the variations as schematically exemplified in FIG. 21A to FIG. 21D, the activity amount processing device 3 may visualize information on the shift of the biorhythm of the test subject, advice on the action capable of adjusting the biorhythm, and the like.

The modes of visualization exemplified in FIGs. 16 to 20 and FIGs. 21A to 21D may be appropriately combined. In addition, the modes of visualization of the information on the shift of the biorhythm of the test subject and the advice on the adjustment of the biorhythm are not limited to the modes exemplified in FIGs. 16 to 20 and FIGs. 21A to 21D.

Any visualization mode may be adopted as long as the mode makes it easy for the test subject to visually recognize the shift of the biorhythm of the test subject and the method for adjusting the shifted biorhythm; additionally, highlighted display, non-highlighted display, and the like may be used in the visualization.

In addition, the information on the action capable of adjusting the biorhythm may be visualized as two or more action candidates. The test subject may select an action likely to achieve the adjustment of his or her biorhythm, from among the visualized two or more action candidates.

The visualization target on which the information according to the above various modes is not limited to the display 11 of the PC 3 or the server 7, and may be the display of the activity amount meter 2 or the display of the mobile terminal 4.

For example, the PC 3, the server 7, or the like may transmit, to the activity amount meter 2, the mobile terminal 4 or the like, signals for displaying information on the shift of the biorhythm of the test subject, advice on the adjustment of the biorhythm, and the like. The signals may include data for controlling the above-described various visualization modes.

In the above-described embodiment, the advice concerning the adjustment of the sleeping time and the wake-up time of the test subject is visualized; however, alternatively or additionally, advice on a nap time recommended to the test subject may be visualized.

The advice on the sleeping time, wake-up time, and nap time of the test subject may be regarded as an example of information for controlling the time of being exposed to light or the time of being not exposed to light so that the test subject may adjust the biorhythm in a self-supporting manner.

As discussed above, according to the above embodiment, by detecting a peak frequency, which corresponds to the characteristic point, from a result obtained through the frequency analysis of a time waveform of activity amount data of a test subject, it is possible to detect a frequency component that is derived from a biorhythm of the test subject and exhibits one cycle in a time period of approximately 24 hours.

In other words, from the time waveform of activity amount data of the test subject, components likely to be affected by the movement following the physical activities of the test subject are removed or suppressed so as to make it possible to detect the frequency component derived from the biorhythm of the test subject.

Accordingly, it is possible to detect the circadian rhythm of the test subject with ease without acquiring the blood and the rectal temperature of the test subject. Therefore, it is possible to easily detect a shift between the circadian rhythm of the test subject and the standard rhythm, and provide the test subject with useful and appropriate comments and advice for improving the shift.

In other words, it is possible to provide, to the test subject, information such as advice on the action to be taken in accordance with the biorhythm of the test subject. As a result, it is easy for the test subject to adjust his or her biorhythm in a self-supporting manner and keep it in a normal state, which will largely contribute to health management or the like of the test subject.

For example, it is possible to give advice on a sleeping time, wake-up time, and the like recommended to the test subject in such a manner as to make the shift of the circadian rhythm smaller. Since it is considered that light is effective for controlling a circadian rhythm, it is also possible to advise the test subject to control the time of exposure to light, the time of non-exposure to light, and the like.

In addition, since the activity amount processing device 3 detects the biorhythm based on the activity amount data of the individual test subject, it is possible to detect the biorhythm of each individual test subject without using an average value or the like of a plurality of test subjects or without rounding a personal error.

Therefore, it is possible to accurately detect the biorhythm of the individual test subject, even in a case of biorhythms which differ significantly depending on types of individual persons, that is, a biorhythm of a so-called morning person, a biorhythm of a so-called night person, and the like. Therefore, it is possible to improve the detection precision of the biorhythm shift of the individual test subject, and improve the accuracy of the advice and the like concerning the biorhythm adjustment of the individual test subject.

In addition, the activity amount meter 2 and the mobile terminal 4 may not be mounted in a mode of being fixed to the test subject in order to measure the activity amount data. For example, in a case where a sensor capable of measuring an amount of activity of the test subject without contact, such as an inertial sensor or a radio wave sensor, is applied to the activity amount sensor 21 (or 41), it is possible to measure the amount of activity in a state in which the test subject mounts the activity amount meter 2 or the mobile terminal 4 on the waist, or has it accommodated in a pocket of the clothes or the like.

In the case where a radio wave sensor is applied to the activity amount sensor 21 (or 41), even when the activity amount meter 2 or the mobile terminal 4 is placed at a location separate from the body of the test subject, it is possible to measure the amount of activity as long as the activity amount meter 2 or the mobile terminal 4 is placed within a range from which it is possible for the radio waves to reach the test subject.

Accordingly, the activity amount meter 2 and the mobile terminal 4 may not necessarily be carried by the test subject. For example, the activity amount meter 2 and the mobile terminal 4 may be placed in a room space where the test subject is positioned.

In any case, because the sensor is not required to be mounted in the mode of being fixed to the test subject (for example, the sensor is directly attached to the skin, or the like), for example, the test subject is not required to bear a physical and a psychological burden at the time of measuring the amount of activity.

Accordingly, even in a case where an amount of activity is measured for a long time, for example, equal to or more than 24 hours, it is possible to detect a circadian rhythm of the test subject with ease without obstructing the daily life of the test subject by the measurement. In other words, usability with respect to the circadian rhythm measurement may be improved.

An amount of activity of a test subject may be measured in such a manner that plural activity amount sensors 21 are temporally combined. The plurality of activity amount sensors 21 may be a combination of different types of sensors, for example, a combination of a radio wave sensor and an inertial sensor.

For example, while the test subject is at home, it is possible to measure an amount of activity of the test subject by a radio wave sensor being placed in a room; while the test subject is out of the house, it is also possible to measure the amount of activity by an inertial sensor of the activity amount meter 2 being carried by the test subject.

As a non-limiting example, it is assumed that the amount of activity is measured by the inertial sensor of the activity amount meter 2 carried by the test subject from 9 a.m. to 6 p.m., and the amount of activity is measured by the radio wave sensor placed in the room from 6 p.m. to 9 a.m. of the next morning.

In this case, the activity amount processing device 3 may couple, in time series, the activity amount data measured by the inertial sensor during the period of time from 9 a.m. to 6 p.m. and the activity amount data measured by the radio wave sensor placed in the room during the period of time from 6 p.m. to 9 a.m. of the next morning, and then may perform the frequency analysis.

In other words, the activity amount data exemplified in FIG. 5 may be considered equivalent to the data in which pieces of activity amount data obtained by the plurality of activity amount sensors 21 are coupled in time series.

Then, the activity amount processing device 3 assumes a single day to be just one second in a pseudo manner, and defines a one-day period to be equivalent to 1 Hz, for example, thereby making it possible to obtain a biorhythm of the test subject close to 1 Hz. The activity amount processing device 3 may obtain a period derived from the biorhythm, from an effectual time during which the activity amount data is measured by both the activity amount sensor 21 carried by the test subject and the radio wave sensor placed in the room.

For the method of obtaining the activity amount data from the measurement value of the radio wave sensor, a concept called "extended wavelength" may be applied. For example, changes in amplitude and frequency appear, in accordance with the magnitude and speed of the movement of the test subject, in a beat signal corresponding to the Doppler effect, which is an output signal of the radio wave sensor.

As described above, since the output signal of the radio wave sensor includes information correlated with the amount of activity of the test subject, it is possible to obtain the amount of activity (which may also be referred to as "exercise strength") of the test subject from the measurement value of the radio wave sensor.

For example, a length of a trajectory depicted by the changes in amplitude and frequency that appear in accordance with the magnitude and speed of the movement of the test subject in a time region is defined as "extended wavelength". In other words, the "extended wavelength" is equivalent to a length of a line segment obtained by linearly extending the time waveform of the measurement value of the radio wave sensor in the time region. Hence, the "extended wavelength" is a concept different from a general "wavelength".

As the amount of activity of a test subject per unit time is larger, the length of the "extended wavelength" per unit time is likely to become longer; in contrast, as the amount of activity of the test subject per unit time is smaller, the "extended wavelength" per unit time is likely to become shorter.

Accordingly, it is possible for the activity amount processing device 3 to obtain the activity amount data of the test subject from the "extended wavelength" by converting the measurement value of the radio wave sensor into the "extended wavelength".

The "extended wavelength" may be illustratively calculated by successively storing measurement values of the radio wave sensor in the storage section 30 (or 70) at a given period (which may be referred to as "sampling period") and adding the amounts of change in amplitude values over a unit time.

Alternatively, the "extended wavelength" may be calculated by a computing formula in which an interval of a certain curved line A-B in a time waveform, which is a measurement value of the radio wave sensor, is divided into a plurality of tiny intervals, each of the tiny intervals is approximated by a line segment, and then lengths of the line segments are added.

The concept of the "extended wavelength" may be applied not only to the measurement value of the radio wave sensor, but also to measurement values of other types of sensors, such as a measurement value of an inertial sensor, a measurement value of a heartbeat sensor, a measurement value of a pulse sensor, and the like as long as these sensors are capable of measuring the amount of activity of a test subject.

### (Others)

In the above embodiments and modifications, although the example in which the target of activity amount data measurement and the target of biorhythm detection are "persons" is described, the target of activity amount data measurement and the target of biorhythm detection may be applied to animals other than "persons".

For example, measuring the amount of activity of a pet animal or an animal in a zoo and detecting the biorhythm thereof with ease, will also be of assistance in health management of animals such as a pet animal or the like.

### Reference Signs List

- 1: INFORMATION PROCESSING SYSTEM
- 2: ACTIVITY AMOUNT METER
- 3: PERSONAL COMPUTER (PC)
- 4: MOBILE TERMINAL
- 5: NETWORK
- 6: ROUTER
- 7: INFORMATION PROCESSING APPARATUS (SERVER)
- 11: DISPLAY
- 12: PRINTER
- 21, 41: ACTIVITY AMOUNT SENSOR
- 22, 31, 42, 71: PROCESSOR
- 23, 32, 43, 72: MEMORY
- 24, 34, 44, 74: COMMUNICATION INTERFACE (IF)
- 25, 36, 45, 76: BUS
- 30, 70: STORAGE SECTION
- 33, 73: STORAGE UNIT
- 35, 75: PERIPHERAL IF

## Claims

1. An activity amount processing device comprising:
an acquisition unit configured to acquire activity amount data corresponding to activity of a subject measured by an activity amount meter; and
a processing unit configured to detect a biorhythm of the subject from a result of frequency analysis of a time waveform of the activity amount data and visualize information on an action recommended to the subject with respect to the detected biorhythm.

2. The activity amount processing device according to claim 1,
wherein the processing unit visualizes information on the action capable of reducing a shift between the biorhythm and a standard rhythm.

3. The activity amount processing device according to claim 2,
wherein the processing unit determines that the biorhythm is ahead of the standard rhythm when the biorhythm is less than a lower limit value of a predetermined range capable of being determined to be normal, and visualizes information on the action capable of delaying the biorhythm.

4. The activity amount processing device according to claim 3,
wherein the information on the action capable of delaying the biorhythm is information indicating that, among a sleeping time and a wake-up time of the subject, the sleeping time is delayed.

5. The activity amount processing device according to any one of claims 2 through 4,
wherein the processing unit determines that the biorhythm is behind the standard rhythm when the biorhythm exceeds an upper limit value of a predetermined range capable of being determined to be normal, and visualizes information on the action capable of accelerating the biorhythm.

6. The activity amount processing device according to claim 5,
wherein the information on the action capable of accelerating the biorhythm is information indicating that, among the sleeping time and the wake-up time of the subject, the wake-up time is made earlier.

7. The activity amount processing device according to any one of claims 2 through 6,
wherein, in a case where the biorhythm falls within a predetermined range capable of being determined to be normal, the processing unit visualizes information indicating that the biorhythm is normal and any action for adjusting the biorhythm is not required.

8. An activity amount processing method comprising:
acquiring activity amount data corresponding to activity of a test subject measured by an activity amount meter;
detecting a biorhythm of the test subject from a result of frequency analysis of a time waveform of the activity amount data; and
visualizing information on an action recommended to the test subject with respect to the detected biorhythm.

9. An activity amount processing program that causes a computer to execute a process, the process comprising:
acquiring activity amount data corresponding to activity of a test subject measured by an activity amount meter;
detecting a biorhythm of the test subject from a result of frequency analysis of a time waveform of the activity amount data; and
visualizing information on an action recommended to the test subject with respect to the detected biorhythm.
